# EUROPÄISCHE PATENTSCHRIFT

(11) **EP 2 826 523 B1**
(45) Veröffentlichungstag und Bekanntmachung des Hinweises auf die Patenterteilung: **19.08.2015**
(21) Anmeldenummer: 14168457.1
(22) Anmeldetag: 15.05.2014
(51) Int. Cl.: A61N 1/365, A61N 1/368, A61N 1/37

(54) **Implantierbares Herztherapiegerät zum Erfassen biventrikulärer Tachykardien.**
Implantable heart therapy device
Appareil thérapeutique cardiaque implantable

(30) Priorität: 17.07.2013 US 201361847093 P; 14.08.2013 US 201361865625 P
(43) Veröffentlichungstag der Anmeldung: 21.01.2015
(73) Patentinhaber: BIOTRONIK SE & Co. KG, 12359 Berlin (DE)
(72) Erfinder: Dörr, Thomas, 12437 Berlin (DE); Kucher, Andreas, 16303 Schwedt (DE)
(74) Vertreter: Galander, Marcus

(56) Entgegenhaltungen:
- US-A1- 2008 269 817
- US-A1- 2009 125 077
- US-A1- 2011 082 512
- US-A1- 2013 030 314
- BAROLD S SERGE ET AL: "Dissimilar ventricular rhythms: implications for ICD therapy", HEART RHYTHM : THE OFFICIAL JOURNAL OF THE HEART RHYTHM SOCIETY, ELSEVIER, USA, Bd. 10, Nr. 4, 1. April 2013 (2013-04-01), Seiten 510-516, XP009178789, ISSN: 1556-3871, DOI: 10.1016/J.HRTHM.2012.12.004

## Beschreibung

Die Erfindung betrifft ein implantierbares biventrikuläres Herztherapiegerät mit einer Therapiegerätsteuerung, die eine Tachykardie-Erkennungseinheit aufweist sowie mit einer rechtsventrikuläre Stimulationseinheit und einer linksventrikuläre Stimulationseinheit, die mit der Therapiegerätsteuerung wirkverbunden sind. Die Therapiegerätsteuerung ist ausgebildet, eine antitachykarde Stimulation (ATP) auszulösen.

Aus EP 1 857 140 A2 ist ein Herztherapiegerät mit einer Stimulationseinheit bekannt, die mit einer Stimulationselektrode zum Stimulieren eines Ventrikels eines Herzens verbunden ist oder verbunden werden kann. Die Stimulationseinheit ist ausgebildet, sowohl Stimulationsimpulse als auch Defibrillationsschocks zu erzeugen und weist hierzu zumindest einen Hochspannungskondensator auf, in dem die für einen Defibrillationsschock nötige elektrische Energie zu speichern ist. Außerdem weist das bekannte Herztherapiegerät einen Detektor auf, der dazu ausgebildet ist, vom Herzen aufgenommene physiologische Signale zu verarbeiten und auf Basis dessen das Vorliegen einer akuten ventrikulären Tachykardie oder Fibrillation zu detektieren. Weiterer Bestandteil des bekannten Herztherapiegerätes ist eine Steuereinheit, die mit dem Detektor und der Stimulationseinheit verbunden ist und die ausgebildet ist, auf ein Ausgangssignal des Detektors anzusprechen und die Stimulationseinheit entweder zur Abgabe einer eine antitachykarde Therapie bildenden Folge von Stimulationsimpulsen oder eines Defibrillationsschocks anzusteuern. Das aus EP 1 857 140 bekannte Herztherapiegerät ist zur Therapie des rechten Ventrikels eines Herzens ausgebildet.

Derartige Herztherapiegeräte werden auch als implantierbare Kardioverter/Defibrillatoren (ICD) bezeichnet. Es handelt sich also bei den hier gemeinten Geräten in erster Linie um implantierbare Herztherapiegeräte, die in der Lage sind, eine Tachykardie eines Herzens zu therapieren.

Mit Tachykardien sollen im Rahmen dieser Anmeldung sowohl Tachykardien im engeren Sinne, die sich durch einen stabilen Herzrhythmus mit pathologisch hoher Frequenz auszeichnen, als auch Fibrillationen gemeint sein. Bekannte Therapien, die durch einen Herzstimulator der eingangs genannten Art abgegeben werden können, sind eine antitachykarde Stimulation oder ein Defibrillationsschock.

Ein Defibrillationsschock ist ein elektrischer Stromstoß, der in das Herz abgegeben wird und der eine ausreichend hohe Spannung und Energie besitzt, um eine von Fibrillation betroffene Herzkammer vollständig zu erregen und damit refraktär zu machen. Auf diese Weise werden für Fibrillationen typische kreisende Erregungen unterbrochen. Im Falle einer Tachykardie im engeren Sinne, die - soweit der Ventrikel betroffen ist - häufig auch als ventrikuläre Tachykardie bezeichnet und mit VT abgekürzt wird (im Unterschied zur ventrikulären Fibrillation VF) ist häufig eine erfolgreiche Therapie auf dem Wege der antitachykarden Stimulation (anti tachyarrhythmia pacing; ATP) möglich. Im Rahmen der antitachykarden Stimulation gibt der Herzstimulator eine Folge von Stimulationsimpulsen aus, deren Energie wesentlich geringer ist, als die Energie eines Defibrillationsschocks und die daher auch nicht schmerzhaft sind. Im Rahmen der antitachykarden Stimulation werden solche Stimulationsimpulse vergleichsweise niedriger Energie mit einer Frequenz abgegeben, die größer ist, als die Frequenz der erfassten Tachykardie. In vielen Fällen kann auf diese Weise eine Tachykardie beendet werden, ohne dass der Patient Schmerzen erleiden muss oder dass der Energiebedarf besonders hoch ist.

Außerdem sind Therapiegeräte zur Abgabe von Stimulationsimpulsen an beide Ventrikel eines Herzens, also den linken (LV) und/oder den rechten Ventrikel (RV) bekannt. Derartige Therapiegeräte werden als biventrikuläre Therapiegeräte bezeichnet.

Der Erfindung liegt die Aufgabe zugrunde, ein biventrikuläres Therapiegerät, das eine hohe Therapieeffizienz der antitachykarden Stimulation (ATP) ermöglicht.

Erfindungsgemäß wird diese Aufgabe durch ein implantierbares biventrikuläres Herztherapiegerät mit einer Therapiegerätsteuerung gelöst, die eine Tachykardie-Erkennungseinheit aufweist, welche wenigstens indirekt mit zumindest einer rechtsventrikulären Wahrnehmungselektrode und zumindest einer linksventrikulären Wahrnehmungselektrode derart verbunden oder zu verbinden ist, dass der Tachykardie-Erkennungseinheit im Betrieb einen zeitlichen Verlauf elektrischer Potentiale im Herzen repräsentierende Signale oder daraus abgeleitete Signale zugeführt werden. Die Tachykardie-Erkennungseinheit ist ausgebildet, das ihr zugeführte Signal und dessen zeitlichen Verlauf auszuwerten und ein Tachyarrhythmiesignal zu generieren, falls das zugeführte Signal vorgegebene Kriterien erfüllt. Dabei ist die Tachykardie-Erkennungseinheit ausgebildet für die Erkennung von ventrikulären Tachykardien simultan die Herzfrequenz an der rechtsventrikulären und an der linksventrikulären Wahrnehmungselektrode zu bewerten.

Das implantierbare biventrikuläre Herztherapiegerät weist außerdem eine rechtsventrikuläre Stimulationseinheit und eine linksventrikuläre Stimulationseinheit auf, die mit der Therapiegerätsteuerung wirkverbunden sind. Die rechtsventrikuläre Stimulationseinheit und die linksventrikuläre Stimulationseinheit sind in Verbindung mit der Therapiegerätsteuerung (ausgebildet, Stimulationsimpulse für eine rechtsventrikuläre und/oder eine linksventrikuläre antitachykarde Therapie (ATP) zu generieren und über wenigstens eine jeweils anzuschließende oder angeschlossene rechts- bzw. linksventrikuläre Stimulationselektrode abzugeben.

Außerdem ist die Tachykardie-Erkennungseinheit ausgebildet, eine dissimilare Tachykardie zu detektieren und zu bestimmen, welche der über die rechtsventrikuläre und über die linksventrikuläre Wahrnehmungselektrode wahrgenommenen Herzfrequenzen größer ist. Desweiteren ist die Therapiegerätsteuerung ausgebildet, eine Abgabe von Stimulationsimpulsen für eine antitachykarde Stimulation über die rechts- oder die linksventrikuläre Stimulationselektrode derart zu steuern, dass die Stimulationsimpulse für eine antitachykarde Stimulation im Betrieb des Herztherapiegerätes über diejenige Stimulationselektrode abgegeben werden, die demselben Ventrikel zugeordnet ist, wie die Wahrnehmungselektrode über die im Falle einer dissimilären Tachykardie die jeweils niedrigere Herzfrequenz erfasst wurde (Frequenzkriterium für die Auswahl des Therapiekanals; Therapiekanal bezeichnet eine einem jeweiligen Ventrikel zugeordnete Stimulationseinheit).

Es ergibt sich somit eine Anordnung zur Optimierung der Therapieeffizienz der antitachykarden Stimulation (ATP) durch die Nutzung biventrikulärer Wahrnehmung zur automatischen Bestimmung des ATP-Stimulationsortes (RV, LV) bei Patienten mit dissimilaren ventrikulären Tachykardien. Das erfindungsgemäße Herztherapiegerät erlaubt es die Effizienz der antitachykarden Stimulation deutlich zu steigern und so gleichzeitig die Notwendigkeit von Defibrillationstherapien zu senken (Schockreduzierung).

Ein erfindungsgemäßes Herztherapiegerät ist beispielsweise ein implantierbarer Defibrillator, an den wenigstens eine rechtsventrikuläre Elektrode und eine linksventrikuläre Elektrode zur Wahrnehmung und Stimulation des Herzens angeschlossen oder anzuschließen ist. Die Elektroden sind dann jeweils mit einer Tachykardieerkennungseinheit verbunden, die so ausgeführt ist, dass für die Erkennung von ventrikulären Tachykardien simultan die Herzfrequenz an der rechts-ventrikulären und an der linksventrikulären Elektrode bewertet wird. Der implantierbare Defibrillator besitzt außerdem eine Einheit zur Abgabe antitachykarder Stimulation an der rechtsventrikulären Elektrode und eine Einheit zur Abgabe antitachykarder Stimulation an der linksventrikulären Elektrode sowie eine Auswerte- und Therapiesteuereinheit (als Teil der Therapiegeräteteuerung), die den Stimulationsort für die antitachykarde Stimulation immer auf die jeweils langsamere Ventrikelseite festlegt, wenn eine dissimilare Tachykardie vorliegt.

Die Erfindung schließt die Erkenntnis ein, dass bisherige marktverfügbare ICD-Systeme ausschließlich mit einem rechtsventrikulärem VT/VF-Erkennungskanal für die Tachykardieerkennung und für die entsprechende Therapieauswahl arbeiten. Der Stimulationsort der ATP ist dabei statisch programmierbar (RV, LV, BiV).

Einige Systeme bieten auch in der VF-Zone einen Versuch einer ATP-Therapie an, der unmittelbar vor oder mit dem Ladebeginn abgegeben wird, allerdings nur dann, wenn der rechtsventrikuläre Rhythmus zu diesem Zeitpunkt ein Frequenz- und/oder Stabilitätskriterium erfüllt ("ATP one shot").

Die Erfinder haben außerdem erkannt, dass die bei den gegenwärtigen ICDs eingesetzte rein rechtsventrikuläre Wahrnehmung den Nachteil hat, dass bei einer sogenannten dissimilaren ventrikulären Tachyarrythmie u.U. eine falsche Therapieauswahl getroffen werden kann. Ist z.B. der Rhythmus im rechten Ventrikel bereits in einer VF-Zone und instabil, der linke Ventrikel jedoch noch stabil, wird die Auswahl eines Defibrillationsschocks einer ATP vorgezogen, obwohl klinische Beobachtungen zeigen, dass eine solche ATP-Abgabe - hier dann im linken Ventrikel - durchaus eine hohe Therapieerfolgsrate haben.

Ein weiterer von den Erfindern erkannter potentieller Nachteil der derzeitigen Systeme ist die ausschließliche Nutzung der rechtsventrikulären Wahrnehmung zur Synchronisation auch einer linksventrikulären ATP. Dies ist möglicherweise auch die Ursache dafür, dass bislang keine klinischen Studien bekannt sind, die eine Überlegenheit einer linksventrikulären ATP zeigen.

Die vorgenannten einmaligen ATP-Versuche in der VF-Zone berücksichtigen nicht den links-ventrikulären Rhythmus und auch nicht eine potentielle Rhythmusregularisierung während der Zeit des Ladens, so dass potentiell wirksame ATP-Versuche nicht abgeben werden.

Demgegenüber erlaubt es das erfindungsgemäße Herztherapiegerät, die Therapieeffizienz der ATP nennenswert zu steigern und so die Zahl der notwendigen Schockabgaben zu reduzieren. Es ist dabei zu beachten, dass eine Analyse von 3-Kammer-ICD-Daten gezeigt hat, dass es einen erheblichen Anteil von Episoden mit sog. dissimilaren ventrikulären Tachykardien gibt und ebenso die Rhythmusverläufe schneller Tachykardien über die Zeit eine Regularisierung erfahren können.

Gemäß einem auch unabhängig von der Auswahl desjenigen Ventrikels für eine ATP, der die niedrigere Herzfrequenz zeigt, zu realisierender Gedanke führt zu einem Therapiegerät mit einer Auswerte- und Therapiesteuereinheit (als Teil der Therapiegeräteteuerung), die während des Ladens der Schockkondensatoren für eine Defibrillationstherapie kontinuierlich einen oder beide Ventrikelkanäle nach einer Regularisierung des Rhythmus "absucht" und im Falle eine Regularisierung (z.B. Stabilitätskriterium) einen ATP-Versuch während des Ladens im jeweiligen Kanal abgibt.

Eine entsprechendes implantierbares Hetztherapiegerät zeichnet sich dadurch aus, dass die rechtsventrikuläre Stimulationseinheit und die linksventrikuläre Stimulationseinheit einen oder mehrere Schockkondensatoren zum Speichern von Energie für einen Defibrillationsschock aufweisen oder mit diesem verbunden sind und die Therapiegerätsteuerung ausgebildet ist, während des Ladens des oder der Schockkondensatorenfür eine Defibrillationstherapie den zeitlichen Verlauf der elektrische Potentiale im Herzen repräsentierende Signale oder die daraus abgeleiteten Signale hinsichtlich einer Regularisierung des durch diese Signale repräsentierten Rhythmus zu analysieren und ggf. eine Regularisierung während des Ladens des oder der Schockkondensatorenzu zu detektieren und daraufhin eine antitachykarde Stimulation über diejenige Stimulationselektrode auszulösen, die demselben Ventrikel zugeordnet ist, wie die Wahrnehmungselektrode, über die die einen regularisierten Rhythmus repräsentierenden Signale aufgenommen wurden.

Auf diese weise kann ggf. noch in letzter Sekunde ein Defibrillationsschock vermieden werden und ggf. auch ein vollständiges Laden der Schockkondensatoren, so dass im Ergebnis Energie gespart werden kann, einen Patienten aber zumindest ein schmerzhafter Defibrillationsschock erspart bleibt..

Vorzugsweise ist die Therapiegerätsteuerung ausgebildet, eine Stabilitätsbewertung des durch die den zeitlichen Verlauf der elektrische Potentiale im Herzen repräsentierende Signale oder die daraus abgeleiteten Signale repräsentierten Rhythmus durchzuführen und eine antitachykarde Stimulation über diejenige Stimulationselektrode auszulösen, die demselben Ventrikel zugeordnet ist, wie die Wahrnehmungselektrode, über die einen stabilen oder stabileren Rhythmus repräsentierende Signale aufgenommen wurden (Stabilitätskriterium für die Auswahl des Therapiekanals).

Die Auswahl eines Therapiekanals (d.h. derjenigen Stimulationseinheit, über die eine ATP abgegeben wird) kann somit nicht nur von der über einen jeweiligen Wahrnehmungskanal (d.h. die dem jeweiligen Ventrikel zugeordnete Wahrnehmungselektrode) erfassten Herzfrequenz, sondern oder alternativ vorrangig von der Stabilität des von der erfassten Signalen repräsentierten Rhythmuses abhängig sein. So kann die Therapiegerätesteuerung ausgebildet sein, eine antitachykarde Stimulation über diejenige Stimulationselektrode auszulösen, die demselben Ventrikel zugeordnet ist, wie die Wahrnehmungselektrode, über die einen stabilen oder stabileren Rhythmus repräsentierende Signale aufgenommen wurden, auch unabhängig davon ob über diese Wahrnehmungselektrode auch einen jeweils langsameren Rhythmus repräsentierende Signale aufgenommen wurden.

Die Therapiegerätesteuerung kann somit ausgebildet sein, vorrangig oder ausschließlich das Frequenzkriterium für die Auswahl des Therapiekanals anzuwenden, oder vorrangig oder ausschließlich das Stabilitätskriterium. Insbesondere ist bevorzugt, wenn die Therapiegerätesteuerung vorrangig das Frequenzkriterium für die Auswahl des Therapiekanals anwendet und zusätzlich das Stabilitätskriterium, beispielsweise in dem Sinne, dass die Therapiegerätesteuerung im Falle einer dissimilaren Tachykardie zunächst bestimmt, über welche Wahrnehmungselektrode die jeweils niedrigere Herzfrequenz erfasst wurde und anschließend überprüft, ob die über diese Wahrnehmungselektrode erfassten Signale einen ausreichend stabilen Rhythmus repräsentieren, insbesondere einen stabileren oder ähnlich stabilen Rhythmus wie die über die jeweils andere Wahrnehmungselektrode aufgenommen Signale.

Weiterhin ist es bevorzugt, wenn die Therapiegerätesteuerung ausgebildet ist insbesondere im Falle einer dissimilaren Tachykardie eine oder mehrere antitachykarde Stimulationen auszulösen und eine Abgabe eines Defibrillationsschocks zu unterdrücken, solange über wenigstens eine der Wahrnehmungselektroden erfasste Signale einen stabilen Rhythmus repräsentieren. D.h. eine oder mehrere ATP-Versuche werden einer Schocktherapie vorgezogen, solange eine der Ventrikelseiten einen stabilen Rhythmus aufweist.

Außerdem ist die Therapiegerätesteuerung vorzugsweise dazu ausgebildet zu bestimmen, ob die über wenigstens eine der Wahrnehmungselektroden Signale einen Rhythmus oberhalb eines programmierbaren Grenzwertes repräsentieren und wenn dies der Fall, nur ein einzelne antitachykarde Stimulation auszulösen und gleichzeitig oder unmittelbar danach einen Ladevorgang von einem Schockkondensator für einen Defibrillationsschock auszulösen. Befindet sich eine der Ventrikelseiten bereits in der VF-Zone bzw. oberhalb eines programmierbaren Grenzwertes, so wird nur ein einzelner ATP-Versuch abgeben und gleichzeitig oder unmittelbar danach der Ladevorgang zur Defibrillation gestartet.

Hierbei ist die Therapiegerätesteuerung vorzugsweise ausgebildet eine Erfolg einer ausgelösten antitachykarden Stimulation während des Ladens der Schockkondensatoren zu überprüfen und für den fall, dass die Therapiegerätesteuerung einen Erfolg einer jeweils ausgelösten antitachykarden Stimulation erfasst, eine Abgabe eines Defibrillationsschocks unterdrückt. Die Erfolgsbewertung dieses ATP-Versuchs erfolgt also vorzugsweise während des Ladens der Schockkondensatoren bzw. vor Schockabgabe (siehe "ATP one shot").

Vorzugsweise ist das implantierbare biventrikuläre Herztherapiegerät ein implantierbarer biventrikulärer Kardiovter/Defibrillator (ICD), der ein metallisches Gehäuse, in dem sich die Therapiegerätesteuerung, die Stimulationseinheiten, die Schockkondensatoren bildende elektrische Komponenten befinden und ein Anschlussgehäuse zum Anschluss von Elektrodenleitungen aufweist, an welchen sich Wahrnehmungs- und Stimulationselektroden befinden..

Die Erfindung soll nun anhand eines Ausführungsbeispiels mit Bezug auf die Figuren näher erläutert werden. Von den Figuren zeigt:
- Figur 1:: ein Beispiel für eine dissimilare ventrikuläre Tachyarrhythmie;
- Figur 2:: einen biventrikulären Herzschrittmacher mit rechtsventrikulärer Defbrillationsschockwendel als implantierbaren Herzstimulator;
- Figur 3:: einige Bestandteile des Herzstimulators aus Figur 2 in Form eines vereinfachten Blockdiagramms;
- Figur 4:: einen biventrikulären Dreikammer-Herzschrittmacher und implantierbaren Kardioverter/Defibrillator (ICD) als implantierbaren Herzstimulator;
- Figur 5:: ein Beispiel einer induzierten dissimilären ventrikulären Fibrillation sowie die entsprechenden, vom Therapiegerät erzeugten Markersignale.
- Figur 6:: einen möglichen Ablaufplan für die Therapiesteuerung bei dissimilaren ventrikulären Tachykardien;
- Figur 7:: ein klinisches Beispiel für eine erfolgreiche Terminierung einer dissimilaren ventrikulären Tachykardie;
- Figur 8:: ein Beispiel einer ATP bei erkannter Regularisierung einer VF.

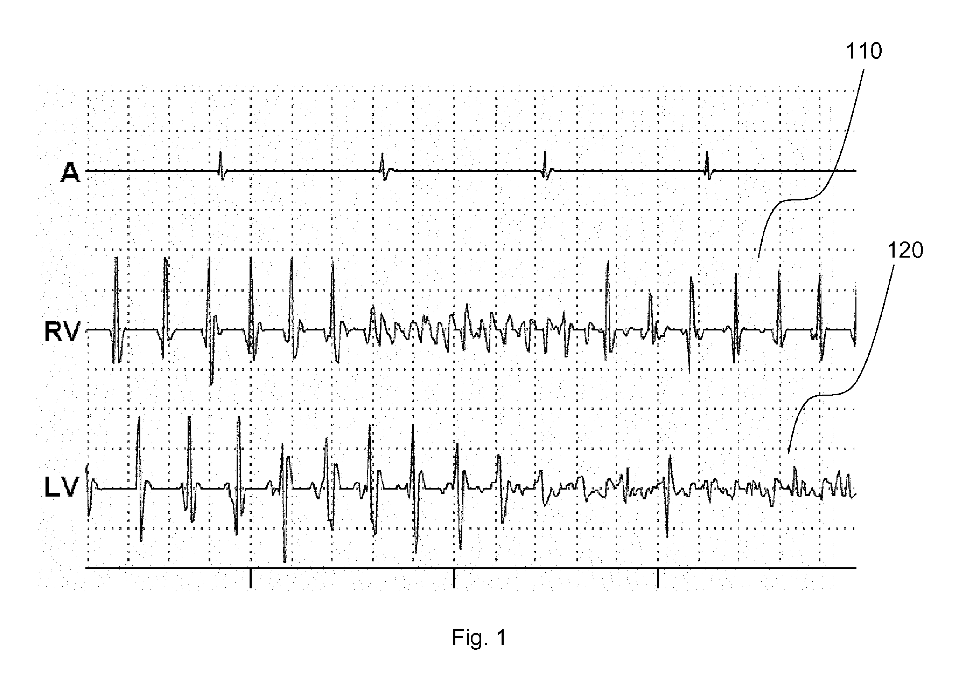

In der Figur 1 ist ein typisches Beispiel einer dissimilaren ventrikulären Tachyarrythmie dargestellt. Hier wechselt im rechten Ventrikel (RV) der Rhythmus von einer stabilen VT über eine kurze Phase von VF auf eine langsamere VT (110), gleichzeitig wechselt zu einem späteren Zeitpunkt der Rhythmus im LV-Kanal von einer stabilen VT zu einem dauerhaften VF (120), dass bei einer rein rechtsventrikulären Detektion nicht wahrgenommen wird und zu einer fehlerhaften Therapieauswahl führt.

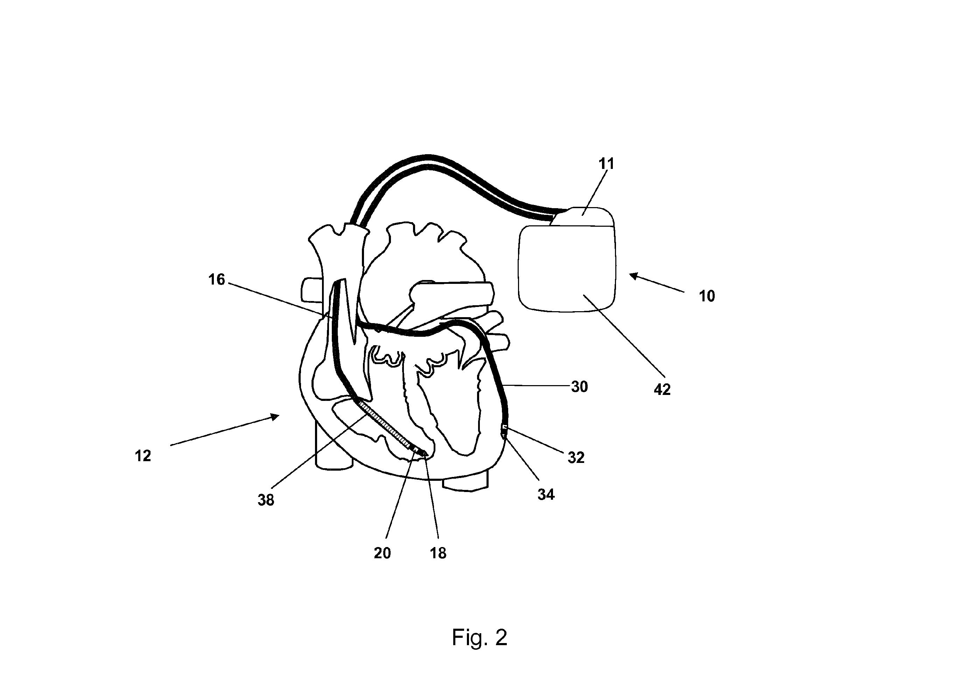

Figur 2 zeigt als Beispiel eines implantierbaren Herztherapiegerätes 10 einen biventrikulären Herzschrittmacher/Defibrillator (ICD oder CRT-D) mit daran angeschlossenen Elektrodenleitungen 16 und 30. Der biventrikuläre Herzschrittmacher 10 ist über die Elektrodenleitungen 16 und 30 mit Stimulations- und Sensingelektroden 18 und 20 bzw. 32 und 34 im rechten bzw. linken Ventrikel eines Herzens verbunden und kann auf diese Weise Stimulationsimpulse an das Herz abgeben und elektrische Potentiale vom Herzen aufnehmen.

Der biventrikuläre Herzschrittmacher 10 besitzt ein metallisches Gehäuse 42 mit einem Anschlussblock oder Header 11 für die Elektrodenleitungen 16 und 30.

Die Elektrodenleitungen 16 und 30 sind über bekannte, standardisierte Steckverbindungen mit Kontaktbuchsen in dem Header (Anschlussgehäuse) 11 des Herzstimulators 10 elektrisch verbunden. Auf diese Weise sind die Elektrodenleitungen 16 und 30 auch mit elektronischen Komponenten im Inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 16 ist eine rechtsventrikuläre Elektrodenleitung und besitzt an ihrem distalen Ende einen rechtsventrikulären Spitzenelektrodenpol RV Tip 18 und in unmittelbarer Nähe einen rechtsventrikulären Ringelektrodenpol RV Ring 20. Beide Elektrodenpole sind im Betrieb im Apex des rechten Ventrikels des Herzens angeordnet und dienen der rechtsventrikulären Wahrnehmung und Stimulation und bilden somit eine Wahrnehmungs- und Stimulationselektrode, wobei die Wahrnehmung in der Regel über Ringelektrodenpol RV Ring 20 und Spitzenelektrodenpol RV Tip 18 als bipolarem Elektrodenpol erfolgt, während die Abgabe von Stimulationsimpulsen über den Spitzenelektrodenpol RV Tip 18 alleine erfolgt. Außerdem trägt die Elektrodenleitung 16 eine rechtsventrikuläre Schockwendel RV Schock 38 als großflächigen Elektrodenpol zur Abgabe von Defibrillationsschocks. Gegenelektrode ist in diesem Fall das Gehäuse 42.

Ferner umfasst das System eine linksventrikuläre Elektrodenleitung 30, die über den Coronar Sinus implantiert wird und ebenfalls einen bipolaren Elektrodenpol für die Wahrnehmung und die Stimulation des linken Ventrikels aufweist.

Die linksventrikuläre Elektrodenleitung 30 weist am distalen Ende eine bipolare Stimulations- und Wahrnehmungselektrode mit einem distalen Spitzenelektrodenpol LV Tip 34 sowie in deren Nähe einem linksventrikulären Ringelektrodenpol LV Ring 32 auf. Die beiden Elektrodenpole LV Tip 34 und LV Ring 32 dienen der rechtsventrikulären Wahrnehmung und Stimulation und bilden somit eine Wahrnehmungs- und Stimulationselektrode, wobei die Wahrnehmung in der Regel über Ringelektrodenpol LV Ring 32 und Spitzenelektrodenpol LV Tip 34 als bipolarem Elektrodenpol erfolgt, während die Abgabe von linksventrikulären Stimulationsimpulsen über den Spitzenelektrodenpol LV Tip 34 alleine erfolgt. Die linksventrikuläre Elektrodenleitung 30 wird vom rechten Atrium 26 des Herzens 12 aus über den Coronarsinus in eine von diesem abzweigende Lateralvene geführt und wird deswegen auch als Coronarsinuselektrodenleitung oder CS-Elektrodenleitung bezeichnet.

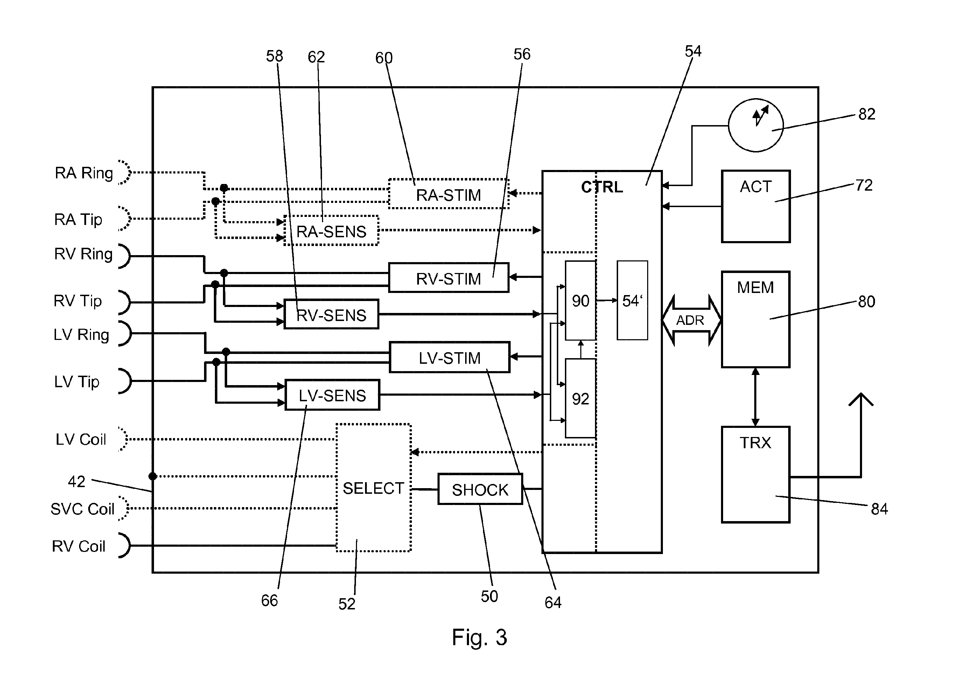

In Figur 3 sind einige der wesentlichen Funktionseinheiten des Herzstimulators 10 dargestellt. In punktierter Liniendarstellung sind außerdem weitere Komponenten dargestellt, wie sie bei einer alternativen Ausführungsvariante eines implantierbaren Herzstimulators zusätzlich vorgesehen sein können.

Auf der linken Seite sind die elektrischen Anschlüsse für die verschiedenen Elektrodenpole 18, 20, 32, 34, und 38 dargestellt. Die Schockelektrode (Schockwendel) 38 ist mit einem Schockimpulsgenerator 50 verbunden. Der Schockimpulsgenerator 50 ist mit einer Steuereinheit 54 verbunden, die den Schockimpulsgenerator 50 bei Bedarf zur Erzeugung und Abgabe eines Kardioversions- oder Defibrillationsschocks ansteuert. In diesem Sinne wirkt die Steuereinheit 54 als Therapiegerätsteuerung 54'. Die Therapiegerätsteuerung 54` ist nur andeutungsweise in Figur 3 eingezeichnet und ist beispielsweise mit dem Schockimpulsgenerator 50 sowie einer rechtsventrikulären Stimulationseinheit 56 und mit einer linksventrikulären Stimulationseinheit 64 verbunden.

Die Steuereinheit 54 umfasst eine Tachykardie-Erkennungseinheit 90 und eine Dislokations-Erkennungseinheit 92.

Der Anschluss für den rechtsventrikulären Spitzenelektrodenpol RV Tip sowie der Anschluss für den rechtsventrikulären Ringelektrodenpol RV Ring sind jeweils sowohl mit der rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Steuereinheit 54 verbunden.

Die rechtsventrikuläre Stimulationseinheit 56 ist dazu ausgebildet, auf ein Ansteuersignal der Steuereinheit 54 hin einen rechtsventrikulären Stimulationsimpuls zu erzeugen und über die Anschlüsse für den rechtsventrikulären Ringelektrodenpol und den rechtsventrikulären Spitzenelektrodenpol abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für den rechtsventrikulären Spitzenelektrodenpol RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung des rechtsventrikulären Spitzenelektrodenpol 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensingeinheit 58 ist dazu ausgebildet, an dem Anschluss für den rechtsventrikulären Ringelektrodenpol RV Ring und den rechtsventrikulären Spitzenelektrodenpol RV Tip anliegende elektrische Potentiale zunächst durch einen Eingangsverstärker zu verstärken und zu filtern. Weiterhin ist die rechtsventrikuläre Sensingeinheit ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R Zacke kennzeichnend für eine natürliche Kontraktion des rechten Ventrikels, die durch Schwellwertvergleich detektiert werden kann. Die rechtsventrikuläre Sensingeinheit 58 gibt daraufhin ein entsprechendes, eine natürliche Kontraktion des rechten Ventrikels anzeigendes Ausgangssignal (z.B. ein Markersignal) an die Steuereinheit 54 und deren Tachykardie-Erkennungseinheit 90 und Dislokations-Erkennungseinheit 92 aus.

In analoger Weise sind auch der Anschluss für den linksventrikulären Spitzenelektrodenpol LV Tip und der Anschluss für den linksventrikulären Ringelektrodenpol LV Ring mit der linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Steuereinheit 54 verbunden. Beide funktionieren ähnlich wie die bereits beschriebenen Stimulationseinheiten 56 und 60 und Sensingeinheiten 58 und 62.

Als weiterer Bestandteil des Herzstimulators 10 ist ein Aktivitätssensor 72 mit der Steuereinheit 54 verbunden. Der Aktivitätssensor 72 ist dazu ausgebildet, ein von der körperlichen Aktivität eines Patienten abhängiges Signal, zum Beispiel ein Bewegungssignal, zu erfassen und ein entsprechendes, die körperliche Aktivität des Patienten anzeigendes Signal an die Steuereinheit 54 auszugeben. Dies erlaubt es, dass die Steuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Steuereinheit 54 verbunden ist und es erlaubt, von der Steuereinheit 54 erzeugte oder ausgewertete Signale zu speichern. Andererseits erlaubt es die Speichereinheit 80, Steuerprogramme für die Steuereinheit 54 in veränderbarer Form zu speichern. Des Weiteren ist die Steuereinheit 54 mit einem Zeitgeber 84 verbunden.

Der Herzstimulator 10 verfügt über mindestens eine bidirektionale Telemetrieschnittstelle 84, um gespeicherte Daten vom elektronischen Implantat an ein externes Gerät 100 übertragen zu können und umgekehrt auch Programmiereinstellungen und Therapiekommandos von diesem externen Gerät 100 empfangen zu können.

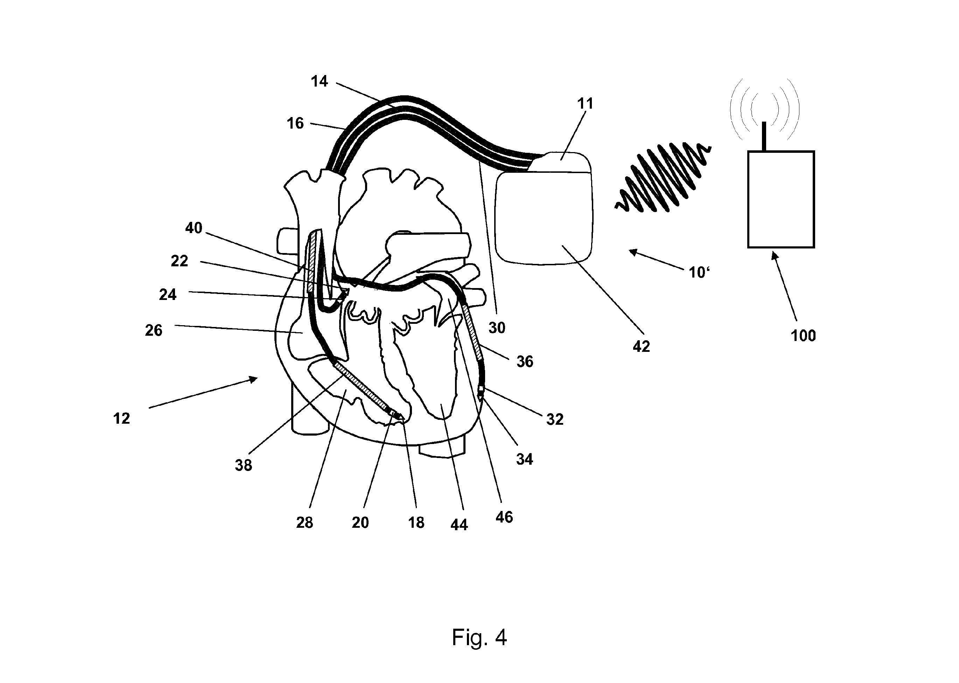

In Figur 4 ist eine alternative Ausführungsvariante eines implantierbaren Herztherapiegerätes in Form eines bi-ventrikulären Dreikammer-Herzstimulators / ICDs 10' dargestellt. Dieser ist über seinen Anschlussblock 11 (Header) mit einer rechtsventrikulären Elektrodenleitung 16, einer linksventrikulären Elektrodenleitung 30 und zusätzlich einer rechtsatrialen Elektrodenleitung 14 verbunden.

Diese Elektrodenleitungen werden im Herzen 12 dauerhaft implantiert. Die rechtsventrikuläre Elektrodenleitung 16 weist dabei am distalen Ende eine bipolare Stimulations- und Wahrnehmungselektrode mit Spitzenelektrodenpol RV Tip 18 und Ringelektrodenpol RV Ring 20 auf. Ferner ist diese Elektrodenleitung mit einer distalen Schockwendel RV Coil 38 und zusätzlich einer proximalen Schockwendel SVC Coil 40 ausgestattet. Die distale Schockwendel RV Coil 38 ist dabei so angeordnet, dass diese im rechten Ventrikel 28 liegt. Die proximale Schockwendel SVC Coil 40 liegt im oberen Teil des rechten Atriums 26 bzw. in der Superior vena cava (oberen Hohlvene).

Die Elektrodenleitung 14 ist eine atriale Elektrodenleitung und weist am distalen Ende eine bipolare Stimulations- und Wahrnehmungselektrode, gebildet von einem Spitzenelektrodenpol RA Tip 22 und einem Ringelektrodenpol RA Ring 24 auf. Die Elektrodenleitung 14 ist im rechten Atrium 26 implantiert.

Bei dem Ausführungsbeispiel in Figur 4 trägt die linksventrikuläre Elektrodenleitung 30 zusätzlich eine linksventrikuläre Schockwendel 36 zur Abgabe von Defibrillationsschocks an den linken Ventrikel. Diese Schockwendel 36 ist dabei so angeordnet, dass diese vom linken Ventrikel 44 bis hinauf zum linken Vorhof 46 reicht. Eine weitere Elektrode für die Schockabgabe stellt das elektrisch aktive Gehäuse 42 des Implantates 10 dar.

Wie Figur 3 anhand der punktiert dargestellten Komponenten zu entnehmen ist, sind der Anschluss für den rechtsatrialen Spitzenelektrodenpol und der Anschluss für den rechtsatrialen Ringelektrodenpol sowohl mit einer rechtsatrialen Stimulationseinheit 60 als auch mit einer rechtsatrialen Sensingeinheit 62 verbunden, die jeweils ihrerseits wiederum mit der Steuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensingeinheit 62 eine entsprechende P-Welle, erzeugt sie ein Ausgangssignal und gibt dieses an die Steuereinheit 54 weiter, welches eine natürliche Kontraktion des rechten Atriums kennzeichnet.

Die bei der in Figur 4 dargestellten Ausführungsvariante vorgesehene linksventrikuläre Schockwendel 36 ist - wie in Figur 3 ebenfalls punktiert dargestellt - über einen Anschluss LV-COIL und eine Elektrodenauswahleinheit 52 ebenfalls mit dem Schockimpulsgenerator 50 verbunden. Mittels der Elektrodenauswahleinheit 52 kann die Steuereinheit 54 zwei oder mehr Elektroden (einschließlich des leitenden Gehäuses 42) auswählen, über die ein Schock abgegeben werden soll.

Bei den in Figuren 2 bis 4 dargestellten Herztherapiegeräten erfolgt die Klassifikation der tachykarden ventrikulären Rhythmusstörungen nun primär über die wahrgenommenen Herzaktionen von der rechtsventrikulären und/oder der linksventrikulären Elektrodenleitung, wobei zum Zeitpunkt einer Therapieauswahl beide Seiten des Ventrikels hinsichtlich der Frequenz und Frequenzstabilität bewertet werden. Unterscheiden sich die Frequenzen beider Ventrikel nennenswert, so wird der langsamere Rhythmus hinsichtlich Frequenz und optional Stabilität bewertet. Eine ATP wird immer an der langsameren Ventrikelseite abgebeben, vorzugsweise nur dann, wenn diese Ventrikelseite eine Frequenz unterhalb einer programmierbaren Frequenzgrenze aufweist oder zumindest eine Stabilität auf dieser Ventrikelseite nachgewiesen werden kann.

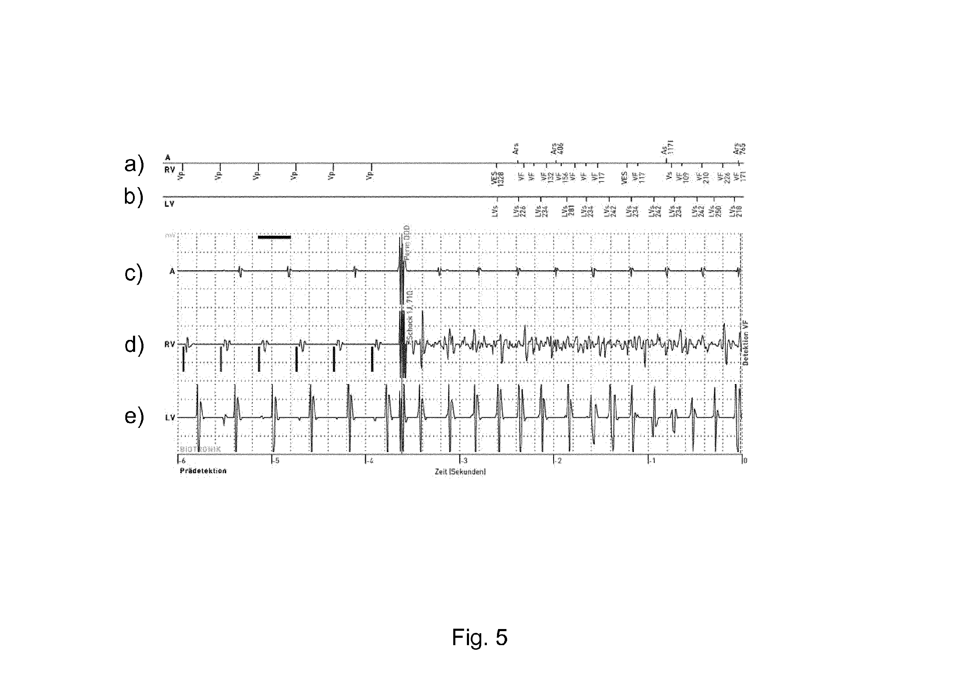

In Figur 5 ist eine typische - in diesem Fall induzierte - dissimilare ventrikuläre Tachykardie dargestellt. Im rechtsventrikulären IEGM (RV) ist ein instabiler und sehr schneller Rhythmus entsprechend eines Kammerflimmerns zu sehen, wobei im linken Ventrikel (LV) noch eine Tachykardie mit verhältnismäßig regelmäßiger Zykluslänge nachgewiesen wird.

Wie im Beispiel dargestellt, wird eine solche dissimilare Tachykardie von herkömmlichen ICD-Systemen als ventrikuläres Flimmern (VF; siehe Zeile "RV") klassifiziert und immer eine Defibrillationsschocktherapie eingeleitet.

Untersuchungen haben jedoch gezeigt, dass eine solche Rhythmusstörung mit einer antitachykarden Stimulation erfolgreich und schmerzfrei behandelt werden kann, wenn die ATP auf der langsameren, stabilen Ventrikelseite appliziert wird. Das elektrophysiologische Erklärungsmodell geht hier von einer noch definierten myokardialen Reizleitungs- und Refraktärzeitstruktur überwiegend im linken Ventrikel aus, obwohl die rechtsventrikuläre Struktur bereits dissoziierte Reizleitungs- und Refraktärzeitbedingungen aufweist. Wird nun die linksseitige VT durch eine ATP erfolgreich beendet, so terminiert auch die Rhythmusstörung auf der rechten Seite mit einer großen Wahrscheinlichkeit.

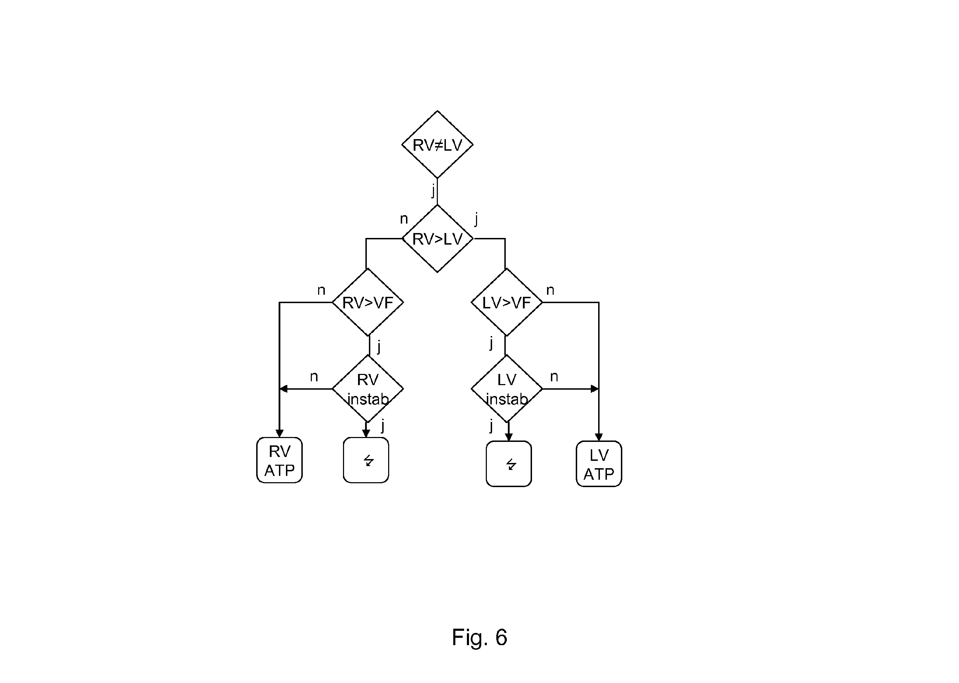

Figur 6 ist ein möglicher Ablaufplan für die Therapiesteuerung bei dissimilaren ventrikulären Tachykardien dargestellt.

Nachdem eine Tachykardie grundsätzlich erkannt wurde, prüft die Therapiesteuereinheit zunächst auf eine Dissimilarität (RV≠LV). Ist der Rhythmus dissimilar, dann wird geprüft, welche Ventrikelfrequenz langsamer ist (RV>LV). Für die jeweils langsamer Seite wird geprüft, ob die Frequenz bereits innerhalb der schockpflichtigen VF-Zone liegt (RV/LV>VF). Ist dies nicht der Fall, wird auf der jeweiligen Ventrikelseite eine ATP abgebeben. Liegt der Rhythmus auch auf der langsameren Ventrikelseite bereits innerhalb der VF-Zone wird immer dann sofort ein Schock ( 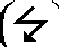 ) abgegeben, wenn der Rhythmus instabil ist (RV/LV instab.), andernfalls wird auch dann ein ATP-Versuch auf der jeweiligen Seite abgegeben und entweder danach oder bereits während der ATP-Abgabe der Ladevorgang für einen Schock gestartet (ATP one shot - hier nicht abgebildet).

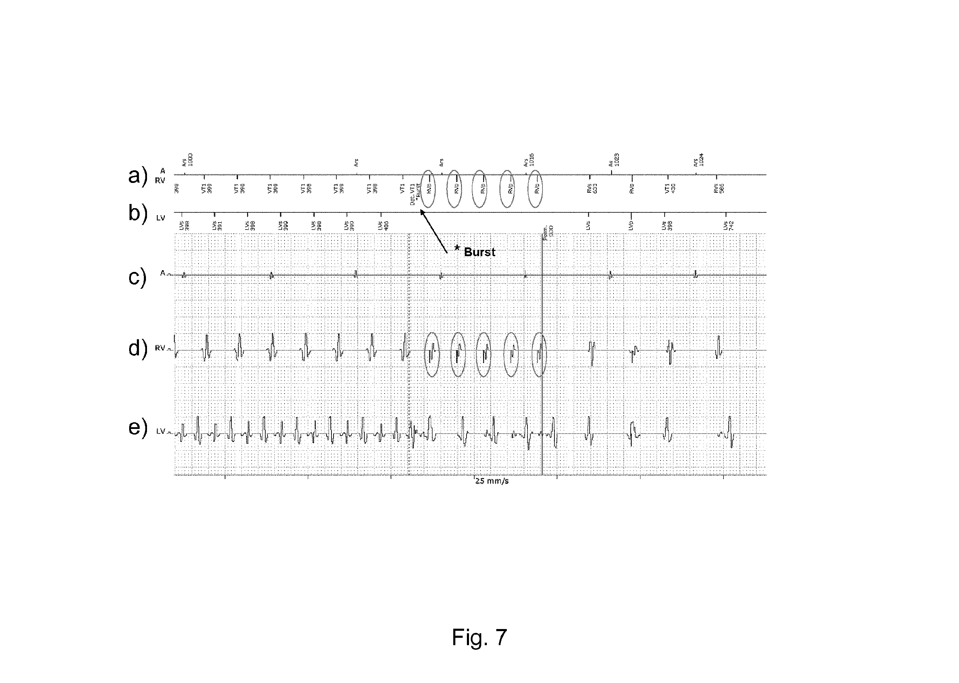

In Figur 7 ist ein klinisches Beispiel für eine erfolgreiche Terminierung einer dissimilaren VT abgebildet. In diesem Beispiel ist die Frequenz der linksventrikuläre Tachykardie doppelt so hoch wie die der rechtsventrikuläre VT und würde eigentlich in die VF-Zone fallen und mit einer Schocktherapie behandelt werden. Dieses Beispiel zeigt hier, dass auch eine ATP (*Burst), abgegebenen auf der langsameren Ventrikelseite (hier RV), die ventrikuläre Tachykardie erfolgreich terminieren kann und eine Schockabgabe nicht notwendig ist.

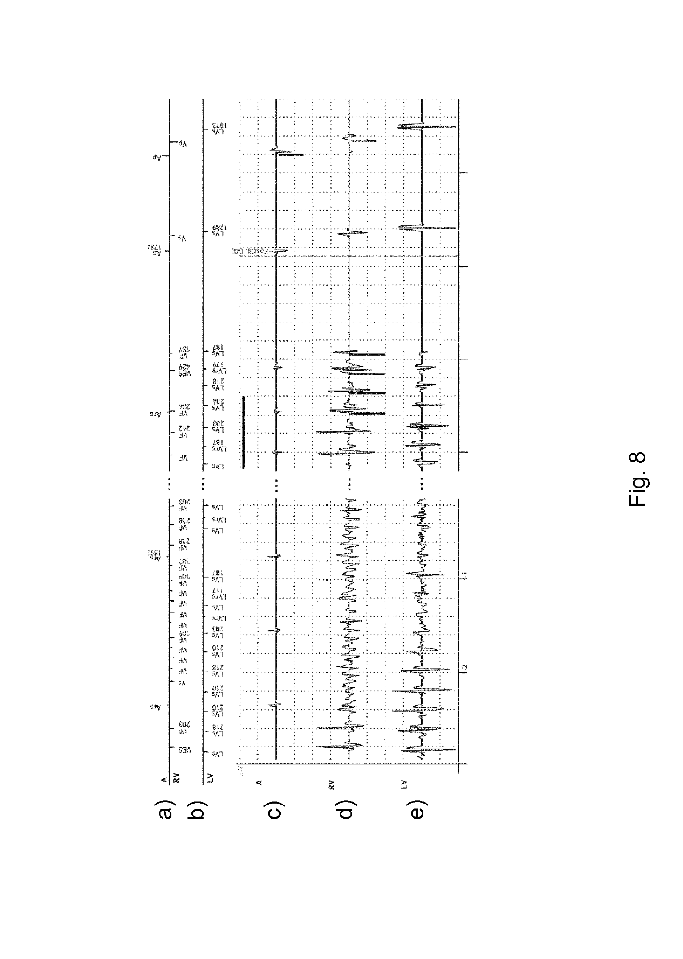

In Figur 8 ist die Funktionsweise eines Herztherapiegerätes illustriert, bei dem die Tachykardie-Erkennungseinheit 90 während des Ladens von Schockkondensatoren für eine Defibrillationstherapie kontinuierlich einen oder beide Ventrikelkanäle nach einer Regularisierung des Rhythmus "absucht" und die Steuereinheit 54 im Falle einer Regularisierung (z.B. Stabilitätskriterium) einen ATP-Versuch während des Ladens im jeweiligen Kanal auslöst. Die Abbildung zeigt ein irreguläres Kammerflimmern, das nach Detektion zum Laden der Schockkondensatoren führt. Während des Ladens der Schockkondensatoren wird der Rhythmus in beiden Kammern auf Regularisierung überprüft und im Falle einer erkannten Regularisierung ein ATP-Versuch in dem zuerst als regulär klassifiziertem Kanal (hier RV) abgegeben. In dem dargestellten Fall ist die ATP erfolgreich und die Schockabgabe wird daher inhibiert.

Als Kriterium für eine Regularisierung dient vorzugsweise ein Stabilitätskriterium, optional mit einer programmierbaren oberen Frequenzgrenze.

Dieses Verfahren lässt sich zur Reduktion nicht notwendiger Schockabgaben auch in einem Einkammersystem anwenden.

Die Erfindung ermöglicht eine Optimierung der Therapieeffizienz der antitachykarden Stimulation, insbesondere bei dissimilaren und bei schnellen ventrikulären Rhythmusstörungen und dient damit der Reduktion der nicht notwendigen Schockabgaben in der ICD-Therapie.

## Patentansprüche

1. Implantierbares biventrikuläres Herztherapiegerät (10) mit einer Therapiegerätsteuerung (54), die eine Tachykardie-Erkennungseinheit (90) aufweist, welche wenigstens indirekt mit zumindest einer rechtsventrikulären Wahrnehmungselektrode (18, 20) und zumindest einer linksventrikulären Wahrnehmungselektrode (32, 34) derart verbunden oder zu verbinden ist, dass der Tachykardie-Erkennungseinheit (90) im Betrieb einen zeitlichen Verlauf elektrischer Potentiale im Herzen repräsentierende Signale oder daraus abgeleitete Signale zugeführt werden, wobei die Tachykardie-Erkennungseinheit (90) ausgebildet ist, ein ihr zugeführtes Signal und dessen zeitlichen Verlauf auszuwerten und ein Tachyarrhythmiesignal zu generieren, falls das zugeführte Signal vorgegebene Kriterien erfüllt, wobei die Tachykardie-Erkennungseinheit (90) ausgebildet ist für die Erkennung von ventrikulären Tachykardien simultan die Herzfrequenz an der rechtsventrikulären und an der linksventrikulären Wahrnehmungselektrode zu bewerten,
wobei das implantierbare biventrikuläre Herztherapiegerät (10) außerdem eine rechtsventrikuläre Stimulationseinheit (56) und eine linksventrikuläre Stimulationseinheit (64) aufweist, die mit der Therapiegerätsteuerung (54) wirkverbunden sind, wobei die rechtsventrikuläre Stimulationseinheit (56) und die linksventrikuläre Stimulationseinheit (64) in Verbindung mit der Therapiegerätsteuerung (54) ausgebildet sind, Stimulationsimpulse für eine rechtsventrikuläre und/oder eine linksventrikuläre antitachykarde Therapie (ATP) zu generieren und über wenigstens eine jeweils anzuschließende oder angeschlossene rechts- bzw. linksventrikuläre Stimulationselektrode (18; 34) abzugeben,
**dadurch gekennzeichnet, dass** die Tachykardie-Erkennungseinheit (90) ausgebildet ist, eine dissimilare Tachykardie zu detektieren und zu bestimmen welches der einen zeitlichen Verlauf elektrischer Potentiale im Herzen repräsentierenden Signale oder daraus abgeleitete Signale eine höhere Herzfrequenz repräsentiert und wobei die Therapiegerätsteuerung (54) ausgebildet ist, eine Abgabe von Stimulationsimpulsen für eine antitachykarde Stimulation über die rechts- oder die linksventrikuläre Stimulationselektrode (18; 34) derart zu steuern, dass die Stimulationsimpulse für eine antitachykarde Stimulation im Betrieb des Herztherapiegerätes (10) über diejenige Stimulationselektrode abgegeben werden, die demselben Ventrikel zugeordnet ist, wie die Wahrnehmungselektrode über die im Falle einer dissimilären Tachykardie ein eine jeweils niedrigere Herzfrequenz repräsentierendes Signal erfasst wurde.

2. Implantierbares biventrikuläres Herztherapiegerät (10) gemäß Anspruch 1, **dadurch gekennzeichnet, dass** die rechtsventrikuläre Stimulationseinheit (56) und die linksventrikuläre Stimulationseinheit (64) einen oder mehrere Schockkondensatoren zum Speichern von Energie für einen Defibrillationsschock aufweisen oder mit diesem verbunden sind und die Therapiegerätsteuerung (54) ausgebildet ist, während des Ladens des oder der Schockkondensatoren für eine Defibrillationstherapie den zeitlichen Verlauf der elektrische Potentiale im Herzen repräsentierende Signale oder die daraus abgeleiteten Signale hinsichtlich einer Regularisierung des durch diese Signale repräsentierten Rhythmus zu analysieren und ggf. eine Regularisierung während des Ladens des oder der Schockkondensatoren zu detektieren und daraufhin eine antitachykarde Stimulation über diejenige Stimulationselektrode (18; 34) auszulösen, die demselben Ventrikel zugeordnet ist, wie die Wahrnehmungselektrode (18, 20; 32, 34), über die die einen regularisierten Rhythmus repräsentierenden Signale aufgenommen wurden.

3. Implantierbares biventrikuläres Herztherapiegerät (10) gemäß Anspruch 1 oder 2, **dadurch gekennzeichnet, dass** die Therapiegerätsteuerung (54) ausgebildet ist, eine Stabilitätsbewertung des durch die den zeitlichen Verlauf der elektrische Potentiale im Herzen repräsentierende Signale oder die daraus abgeleiteten Signale repräsentierten Rhythmus durchzuführen und eine antitachykarde Stimulation über diejenige Stimulationselektrode auszulösen, die demselben Ventrikel zugeordnet ist, wie die Wahrnehmungselektrode (18, 20; 32, 34), über die einen stabilen oder stabileren Rhythmus repräsentierende Signale aufgenommen wurden.

4. Implantierbares biventrikuläres Herztherapiegerät (10) gemäß eines oder mehrerer der Ansprüche 1 bis 3, **dadurch gekennzeichnet, dass** die Therapiegerätesteuerung (54) ausgebildet ist im Falle einer dissimilaren Tachykardie zunächst zu bestimmen, über welche Wahrnehmungselektrode die jeweils niedrigere Herzfrequenz erfasst wurde und anschließend zu überprüfen, ob die über diese Wahrnehmungselektrode erfassten Signale einen ausreichend stabilen Rhythmus repräsentieren, insbesondere einen stabileren oder ähnlich stabilen Rhythmus wie die über die jeweils andere Wahrnehmungselektrode aufgenommen Signale und eine Abgabe von Stimulationsimpulsen für eine antitachykarde Stimulation über diejenige Stimulationselektrode auszulösen, die demselben Ventrikel zugeordnet ist, wie die Wahrnehmungselektrode über die im Falle einer dissimilären Tachykardie ein eine jeweils niedrigere Herzfrequenz repräsentierendes Signal erfasst wurde, sofern dieses Signal einen ausreichend stabilen Rhythmus repräsentiert..

5. Implantierbares biventrikuläres Herztherapiegerät (10) gemäß eines oder mehrerer der Ansprüche 1 bis 4, **dadurch gekennzeichnet, dass** die Therapiegerätesteuerung (54) ausgebildet ist eine oder mehrere antitachykarde Stimulationen auszulösen und eine Abgabe eines Defibrillationsschocks zu unterdrücken, solange über wenigstens eine der Wahrnehmungselektroden erfasste Signale einen stabilen Rhythmus repräsentieren.

6. Implantierbares biventrikuläres Herztherapiegerät (10) gemäß eines oder mehrerer der Ansprüche 1 bis 4, **dadurch gekennzeichnet, dass** die Therapiegerätesteuerung (54) ausgebildet ist zu bestimmen, ob die über wenigstens eine der Wahrnehmungselektroden Signale einen Rhythmus oberhalb eines programmierbaren Grenzwertes repräsentieren und wenn dies der Fall, nur ein einzelne antitachykarde Stimulation auszulösen und gleichzeitig oder unmittelbar danach einen Ladevorgang von einem Schockkondensator für einen Defibrillationsschock auszulösen.

7. Implantierbares biventrikuläres Herztherapiegerät (10) gemäß Anspruch 6, **dadurch gekennzeichnet, dass** der programmierbare Grenzwert eine Herzfrequenz repräsentiert, ab der eine Tachykardie als Fibrillation betrachtet wird.

8. Implantierbares biventrikuläres Herztherapiegerät (10) gemäß Anspruch 6 oder 7, **dadurch gekennzeichnet, dass** die Therapiegerätesteuerung (54) ausgebildet ist einen Erfolg einer ausgelösten antitachykarden Stimulation während des Ladens der Schockkondensatoren zu überprüfen und für den Fall, dass die Therapiegerätesteuerung (54) einen Erfolg einer jeweils ausgelösten antitachykarden Stimulation erfasst, eine Abgabe eines Defibrillationsschocks unterdrückt.

9. Implantierbares biventrikuläres Herztherapiegerät (10) gemäß eines oder mehrerer der Ansprüche 1 bis 8, **dadurch gekennzeichnet, dass** das implantierbare biventrikuläre Herztherapiegerät (10) ein implantierbarer biventrikulärer Kardioverter/Defibrillator (ICD) ist, der ein metallisches Gehäuse, in dem sich die Therapiegerätesteuerung, die Stimulationseinheiten, die Schockkondensatoren bildende elektrische Komponenten befinden und ein Anschlussgehäuse zum Anschluss von Elektrodenleitungen, an welchen sich Wahrnehmungs- und Stimulationselektroden befinden.

## Claims

1. An implantable biventricular cardiac therapy device (10) comprising a therapy device control (54), which has a tachycardia detection unit (90), which is connected or can be connected, at least indirectly, to at least one right-ventricular sensing electrode (18, 20) and at least one left-ventricular sensing electrode (32, 34) such that, during operation, the tachycardia detection unit (90) is fed signals representing a course of electrical potentials in the heart over time or signals derived therefrom, wherein the tachycardia detection unit (90) is designed to evaluate the signal fed thereto and the course thereof over time and to generate a tachyarrhythmia signal if the fed signal meets predetermined criteria, wherein the tachycardia detection unit (90) is designed to evaluate the heart rate at the right-ventricular and at the left-ventricular sensing electrodes simultaneously in order to detect ventricular tachycardias,
wherein the implantable biventricular cardiac therapy device (10) also comprises a right-ventricular stimulation unit (56) and a left-ventricular stimulation unit (64), which are operative connected to the therapy device control (54), wherein the right-ventricular stimulation unit (56) and the left-ventricular stimulation unit (64) are designed, in combination with the therapy device control (54), to generate stimulation pulses for a right-ventricular and/or a left-ventricular anti-tachycardiac therapy (ATP) and to deliver this via at least one right-ventricular or left-ventricular stimulation electrode (18; 34), respectively, which is intended to be connected or is connected,
**characterized in that** the tachycardia detection unit (90) is designed to detect a dissimilar tachycardia and to determine which of the signals representing a course of electrical potentials in the heart over time, or signals derived therefrom, represents a higher heart rate, and wherein the therapy device control (54) is designed to control a delivery of stimulation pulses for an anti-tachycardiac stimulation via the right-ventricular or the left-ventricular stimulation electrode (18; 34) such that, during operation of the cardiac therapy device (10), the stimulation pulses for an anti-tachycardiac stimulation are delivered via the stimulation electrode that is assigned to the same ventricle as the sensing electrode via which, in the case of a dissimilar tachycardia, a signal representing a respectively lower heart rate was detected.

2. The implantable biventricular cardiac therapy device (10) according to claim 1, **characterized in that** the right-ventricular stimulation unit (56) and the left-ventricular stimulation unit (64) comprise one or more shock capacitors for storing energy for a defibrillation shock, or are connected thereto, and the therapy device control (54) is designed to analyze - during the charging of the shock capacitor or the shock capacitors for a defibrillation therapy - signals representing the course of the electrical potentials in the heart over time, or the signals derived therefrom, with respect to a regularization of the rhythm represented by these signals and, optionally, to detect a regularization during the charging of the shock capacitor or the shock capacitors and to then trigger an anti-tachycardiac stimulation via the stimulation electrode (18; 34) that is assigned to the same ventricle as the sensing electrode (18, 20; 32, 34) via which the signals representing a regularized rhythm were recorded.

3. The implantable biventricular cardiac therapy device (10) according to claim 1 or 2, **characterized in that** the therapy device control (54) is designed to carry out a stability evaluation of the rhythm represented by the signals representing the course of the electrical potentials in the heart over time, or signals derived therefrom, and to trigger an anti-tachycardiac stimulation via the stimulation electrode that is assigned to the same ventricle as the sensing electrode (18, 20; 32, 34) via which signals representing a stable or more stable rhythm were recorded.

4. The implantable biventricular cardiac therapy device (10) according to one or more of claims 1 to 3, **characterized in that** the therapy device control (54) is designed to initially determine, in the event of a dissimilar tachycardia, which sensing electrode was used to detect the respectively lower heart rate and then check to determine whether the signals detected via this sensing electrode represent a sufficiently stable rhythm, in particular a rhythm that is more stable than or as stable as the signals recorded via the respective other sensing electrode, and to trigger a delivery of stimulation pulses for an anti-tachycardiac stimulation via the stimulation electrode that is assigned to the same ventricle as the sensing electrode via which, in the case of a dissimilar tachycardia, a signal representing a respectively lower heart rate was detected, provided this signal represents a sufficiently stable rhythm.

5. The implantable biventricular cardiac therapy device (10) according to one or more of claims 1 to 4, **characterized in that** the therapy device control (54) is designed to trigger one or more anti-tachycardiac stimulations and to suppress a delivery of a defibrillation shock, provided signals detected via at least one of the sensing electrodes represent a stable rhythm.

6. The implantable biventricular cardiac therapy device (10) according to one or more of claims 1 to 4, **characterized in that** the therapy device control (54) is preferably designed to determine whether the signals recorded via at least one of the sensing electrodes represent a rhythm above a programmable limit value and, if this is the case, said therapy device control triggers only one anti-tachycardiac stimulation and, simultaneously therewith or immediately thereafter, triggers a procedure to charge a shock capacitor for a defibrillation shock.

7. The implantable biventricular cardiac therapy device (10) according to claim 6, **characterized in that** the programmable limit value represents a heart rate, starting at which a tachycardia is considered to be fibrillation.

8. The implantable biventricular cardiac therapy device (10) according to claim 6 or 7, **characterized in that** the therapy device control (54) is preferably designed to check a success of a triggered anti-tachycardiac stimulation during the charging of the shock capacitors and, in the event that the therapy device control (54) detects a success of a respectively triggered anti-tachycardiac stimulation, suppresses a delivery of a defibrillation shock.

9. The implantable biventricular cardiac therapy device (10) according to one or more of the claims 1 to 8, **characterized in that** the implantable biventricular cardiac therapy device (10) is an implantable biventricular cardioverter/defibrillator (ICD), which comprises a metallic housing, in which electrical components forming the therapy device control, the stimulation units, and the shock capacitors are located, and a connection housing for the connection of electrode leads, at which sensing and stimulation electrodes are located.

## Revendications

1. Appareil de thérapie cardiaque (10) biventriculaire implantable avec une commande d'appareil de thérapie (54), qui présente une unité de reconnaissance de tachycardie (90), laquelle est reliée, ou peut être reliée, au moins indirectement avec au moins une électrode de reconnaissance du ventricule droit (18, 20) et au moins une électrode de reconnaissance du ventricule gauche (32, 34) de telle sorte que les signaux représentant des potentiels électriques dans le coeur au cours du temps, ou les signaux qui en dérivent, sont dirigés vers l'unité de reconnaissance de la tachycardie (90) en fonctionnement, où l'unité de reconnaissance de tachycardie (90) est conçue pour évaluer un signal lui étant transmis et son évolution dans le temps et pour générer un signal de tachyarythmie dans le cas où le signal transmis satisfait à des critères prédéterminés, où l'unité de reconnaissance de tachycardie (90) est conçue pour la reconnaissance de tachycardies ventriculaires et pour évaluer simultanément la fréquence cardiaque sur l'électrode de reconnaissance du ventricule droit et du ventricule gauche,
où l'appareil de thérapie cardiaque (10) biventriculaire implantable présente en outre une unité de stimulation ventriculaire droite (56) et une unité de stimulation ventriculaire gauche (64), qui sont reliées fonctionnellement avec la commande de l'appareil de thérapie 54), où l'unité de stimulation ventriculaire droite (56) et l'unité de stimulation ventriculaire gauche (64) sont conçues en liaison avec la commande d'appareil de thérapie (54) pour générer des impulsions de stimulation pour une thérapie d'anti-tachycardie ventriculaire droite et/ou ventriculaire gauche (ATP) et pour les délivrer par au moins une électrode de stimulation (18 ; 34) ventriculaire droite, respectivement, gauche, à raccorder ou raccordée,
**caractérisé en ce que** l'unité de reconnaissance de tachycardie (90) est conçue pour détecter une tachycardie dissociée et pour déterminer quels signaux représentant les potentiels électriques dans le coeur dans le temps, ou les signaux en étant dérivés, représentent une fréquence cardiaque plus élevée, et où la commande de l'appareil de thérapie (54) est conçue pour commander une libération d'impulsions de stimulation pour une stimulation d'anti-tachycardie par l'électrode de stimulation ventriculaire droite ou gauche (18 ; 34) de telle sorte que les impulsions de stimulation sont délivrées pour une stimulation d'anti-tachycardie en fonctionnement de l'appareil de thérapie cardiaque (10) par l'électrode de stimulation qui est adjointe au ventricule en question, comme l'électrode de reconnaissance, par laquelle un signal représentant une fréquence cardiaque respectivement plus faible a été détectée dans le cas d'une tachycardie dissociée.

2. Appareil de thérapie cardiaque (10) biventriculaire implantable selon la revendication 1, **caractérisé en ce que** l'unité de stimulation ventriculaire droite (56) et l'unité de stimulation ventriculaire gauche (64) présentent un ou plusieurs condensateurs de choc pour le stockage d'énergie en vue d'un choc de défibrillation, ou sont reliées avec celui-ci, et que la commande d'appareil de thérapie (54) est conçue pour analyser les signaux représentant les potentiels électriques dans le coeur au cours du temps, ou les signaux qui en sont dérivés, pour une thérapie de défibrillation pendant la charge du ou des condensateurs de choc, en ce qui concerne une régularisation du rythme représenté par ces signaux et éventuellement, pour détecter une régularisation pendant la charge du ou des condensateurs de choc et déclencher une stimulation anti-tachycardique suite à cela par l'intermédiaire de l'électrode de stimulation (18 ; 34) concernée, par laquelle des signaux représentant un rythme régularisé a été décelé.

3. Appareil de thérapie cardiaque (10) biventriculaire implantable selon les revendications 1 ou 2, **caractérisé en ce que** la commande d'appareil de thérapie (54) est conçue pour exécuter une évaluation de la stabilité des signaux représentant les potentiels électriques dans le coeur au cours du temps, ou des signaux en étant dérivés, représentant le rythme, et pour déclencher une stimulation anti-tachycardique par l'électrode de stimulation concernée qui est adjointe au même ventricule, telle que l'électrode de reconnaissance (18, 20 ; 32, 34), par laquelle on a détecté les signaux représentant un rythme stable ou plus stable.

4. Appareil de thérapie cardiaque (10) biventriculaire implantable selon l'une ou plusieurs parmi les revendications 1 à 3, **caractérisé en ce que** la commande d'appareil de thérapie (54) est conçue pour déterminer d'abord un cas de tachycardie dissociée, par laquelle électrode de reconnaissance on a détecté la fréquence cardiaque respectivement la plus faible et pour ensuite vérifier si les signaux saisis par cette électrode de reconnaissance représentent un rythme suffisamment stable, notamment, un rythme plus stable ou semblable à un rythme stable, comme les signaux saisis par respectivement l'autre électrode de reconnaissance, et pour déclencher une libération d'impulsions de stimulation pour une stimulation anti-tachycardique par l'électrode de stimulation correspondante adjointe au même ventricule, comme l'électrode de reconnaissance, par laquelle un signal représentant une fréquence cardiaque respectivement plus faible a été saisi dans le cas d'une tachycardie dissociée, dans la mesure où ce signal représente un rythme suffisamment stable.

5. Appareil de thérapie cardiaque (10) biventriculaire implantable selon l'une ou plusieurs parmi les revendications 1 à 4, **caractérisé en ce que** la commande d'appareil de thérapie (54) est conçue pour libérer une ou plusieurs stimulations anti-tachycardiques et pour supprimer la libération d'un choc de défibrillation tant que les signaux saisis par au moins l'une des électrodes de reconnaissance représentent un rythme stable.

6. Appareil de thérapie cardiaque (10) biventriculaire implantable selon l'une ou plusieurs parmi les revendications 1 à 4, **caractérisé en ce que** la commande d'appareil de thérapie (54) est conçue pour déterminer si les signaux de l'une des électrodes de reconnaissance représentent un rythme au dessus d'une valeur limite pouvant être programmée et, lorsque c'est le cas, ne déclencher qu'une stimulation anti-tachycardique unique et déclencher au même moment, ou immédiatement après, un processus de charge d'un condensateur de choc pour un choc de défibrillation.

7. Appareil de thérapie cardiaque (10) biventriculaire implantable selon la revendication 6, **caractérisé en ce que** la valeur limite programmable représente une fréquence cardiaque à partir de laquelle une tachycardie est considérée comme une fibrillation.

8. Appareil de thérapie cardiaque (10) biventriculaire implantable selon les revendications 6 ou 7, **caractérisé en ce que** la commande d'appareil de thérapie (54) est conçue pour vérifier l'efficacité d'une stimulation anti-tachycardique déclenchée pendant la charge des condensateurs de choc et pour supprimer la libération d'un choc de défibrillation dans le cas où la commande d'appareil de thérapie (54) a décelé l'efficacité d'une stimulation anti-tachycardique déjà déclenchée.

9. Appareil de thérapie cardiaque (10) biventriculaire implantable selon l'une ou plusieurs parmi les revendications 1 à 8, **caractérisé en ce que** l'appareil de thérapie cardiaque (10) biventriculaire implantable est un défibrillateur automatique implantable (ICD), qui présente un boîtier métallique, dans lequel se trouvent la commande d'appareil de thérapie, les unités de stimulation, les composants électriques formant les condensateurs de choc, et un boîtier de raccordement pour la connexion de lignes d'électrodes sur lesquelles se trouvent les électrodes de reconnaissance et les électrodes de stimulation.
